# EUROPEAN PATENT SPECIFICATION

(11) **EP 4 590 631 B1**
(45) Date of publication and mention of the grant of the patent: **18.03.2026**
(21) Application number: 23769302.3
(22) Date of filing: 20.09.2023
(51) Int. Cl.: C01B 21/086, C01B 21/093, H01M 10/0568, C07C 303/34

(54) **METHOD FOR MANUFACTURING BIS(HALOGENO SULFONYL)IMIDE**
VERFAHREN ZUR HERSTELLUNG VON BIS(HALOGENSULFONYL)IMID
PROCÉDÉ DE FABRICATION DE BIS(HALOGÉNOSULFONYL)IMIDE

(30) Priority: 22.09.2022 EP 22306396
(43) Date of publication of application: 30.07.2025
(73) Proprietor: SPECIALTY OPERATIONS FRANCE, 69003 Lyon (FR)
(72) Inventor: BATT, Frédéric, 69190 Saint-Fons (FR); DERRIEN, Elie, 69530 Brignais (FR); SCHANEN, Vincent, 69006 Lyon (FR); EVANO, Gwilherm, 1050 Ixelles (BE); THEUNISSEN, Cédric, 1050 Bruxelles (BE); ZAKARDJIAN, Agathe, 69190 Saint-Fons (FR)
(74) Representative: Viola, Laura Maria
(86) International application number: PCT/EP2023/075912
(87) International publication number: WO 2024/061955

(56) References cited:
- EP-A1- 2 415 757
- WO-A1-2017/169874
- CN-A- 107 986 248

## Description

### Technical field

The present invention relates to a new synthetic pathway for manufacturing bis(halogeno sulfonyl)imide, which are useful intermediates in the synthesis of lithium bis(fluorosulfonyl)imide (LiFSI).

### Background

Bis(fluorosulfonyl)imide and salts thereof, in particular the lithium salt of bis(fluorosulfonyl)imide (LiFSI), are useful compounds in a variety of technical fields, including in battery electrolytes.

Several methods for the manufacture of LiFSI have been described in the art. Among the various technologies described, the majority uses a fluorination reaction with a fluorinating agent in a solvent.

Many efforts have also been taken in the art to improve the manufacturing methods of the intermediate compounds leading to LiFSI, in particular with regard to purity, yield and cost reduction.

US 2014/0075746 (in the name of Arkema France) discloses a process for the preparation of a bis(sulphonato)imide salt of formula:

(III) (SO₃⁻)- N⁻- (SO₃⁻) 3C⁺

wherein C⁺ represents a monovalent cation,
the process comprising the reaction of amido-sulphoric acid of formula:

   (I) HO-SO₂-NH₂
with a halo-sulphonic acid of formula:

   (II) HO-SO₂-X
wherein X represents a halon atom,
and comprising a reaction with a base which is a salt formed with cation C⁺.

According to an embodiment of such a process, the reaction between compounds of formula (I) and (II) is conducted in the presence of a first base, to provide a bis(sulphonyl)imide of formula :

(IV) HO-SO₂-NH-SO₂-OH

which is then reacted with a second base, which is a salt formed with cation C⁺ to provide the compound of formula (III) above. The compound of formula (III) thus obtained is further purified in water or other polar solvents, such as alcohols.

EP 2415757 (in the name of CENTRAL GLASS CO., LTD.) discloses the reaction between halogenated sulfuryl (such as XSO₂X with X being Cl or F) or halogenated phosphoryl with ammonia in the presence of an organic base (B) to produce B-XSI structures. Comparative examples 1 and 2 respectively disclose the reaction between SO₂F₂ and SO₂Cl₂ and NH₃ without any added base. Only the sulfamide compound was isolated and the conclusion was that the target ammonium salt was hardly obtained in the absence of the organic base.

US 8,840,856 B2 (in the name of CENTRAL GLASS CO., Ltd.) claims a method for producing an imide salt, which comprises the steps of: reacting an alkali metal fluoride, a sulfuryl dihalide or phosphoryl halide and ammonia or an ammonium salt. The presence of the alkali metal fluoride is mandatory and in the examples it is used in molar excess to ammonia. According to comparative example 1, the reaction performed in the absence of the alkali metal fluoride did not provide the desired product but sulfamide was obtained as the primary component.

EP 2920147 B1 (to TRINAPCO Inc.) claims a process for producing a salt of bis(fluorosulfonyl)imide anion, comprising: adding sulfuryl fluoride to solid ammonium fluoride in a solvent in the presence of an aprotic base and isolating the resulting salt. In comparative example 1, NH₄Cl is used in combination with 1,3-bis (dimethylamino)propane (TMPDA) base. However, no disclosure is made of the products thus obtained, it was only described that a mixture is formed.

US 2012/0245386 (to TRINAPCO Inc.) discloses a method comparison adding NH₃ to a SO₂F₂ solution in the presence of an organic base, preferably selected from N,N,N',N'-tetramethyl-1,2-ethanediamine, N,N,N',N'-tetramethyl -1,3-propanediamine and combinations thereof.

WO 2007/104144 (in the name of TRANSFERT PLUS , S.E.C.) discloses the reaction between a compound of formula R₁-SO₂-R₁ and R₆-N⁻ -R₆ Q⁺, wherein each of said R₁ is independently F or Cl, each of R₆ is H, Li, Na, K, Cs or (R₇)₃Si- and Q⁺ is chosen from:

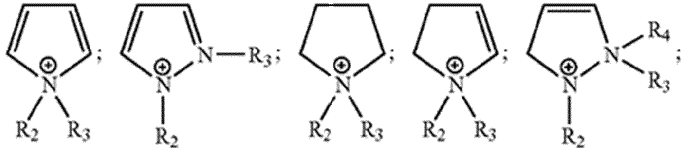

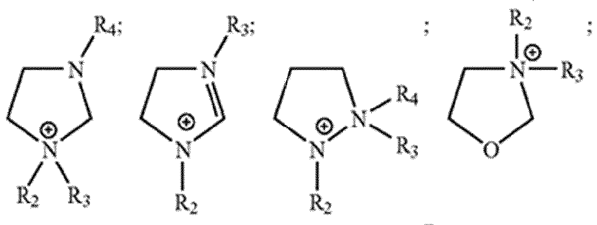

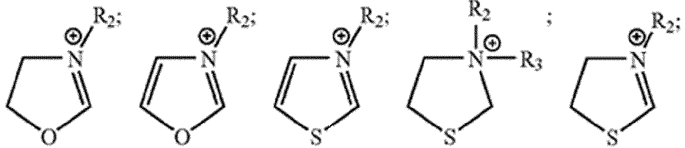

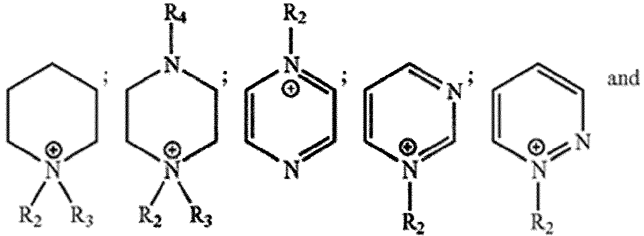

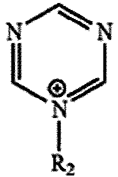

The Applicant is not aware of any disclosure of the preparation of NH₄XSI (with X being Cl or F) starting from an ammonium salt, without the use of a base in a stoichiometric amount. Further methods for producing bis(fluorosulfonyl)imide compounds are known from WO2017/169874 A1 and CN107986248 A.

### Summary of the invention

The Applicant is aware that despite all the attempts in the art, the need is still felt for manufacturing intermediate compounds suitable in the synthesis of lithium bis(fluoro sulfonyl)imide (LiFSI), via processes that can be easily implemented on industrial scale.

In particular, the Applicant faced the problem of manufacturing an intermediate suitable in the synthesis of LiFSI, via a process that satisfies all the above requirements.

The Applicant surprisingly found that a bis(halogeno sulfonyl)imide can be obtained by reacting a sulfuryl halogenide with at least one ammonium compound.

Such a process can be advantageously performed in the absence of any base, which makes the process less complex and with a decreased solid waste generation compared to the processes of the prior art.

The process according to the present invention is also advantageous because the reactants and part of the side products generated during the reaction can be recycled, thus lowering the environmental factor (E factor) of the process, which is defined as the ratio between the "mass of total waste" and "mass of product".

Further to the above, the reaction according to the present invention can be easily controlled, to limit both exothermic reactions and side reactions.

### Disclosure of the invention

In the present application:
- any description, even though described in relation to a specific embodiment, is applicable to and interchangeable with other embodiments of the present invention;
- where an element or component is said to be included in and/or selected from a list of recited elements or components, it should be understood that in related embodiments explicitly contemplated here, the element or component can also be any one of the individual recited elements or components, or can also be selected from a group consisting of any two or more of the explicitly listed elements or components; any element or component recited in a list of elements or components may be omitted from such list.

In a first aspect, the present invention relates to a method for manufacturing bis(halogeno sulfonyl)imide of formula (I) or (II): 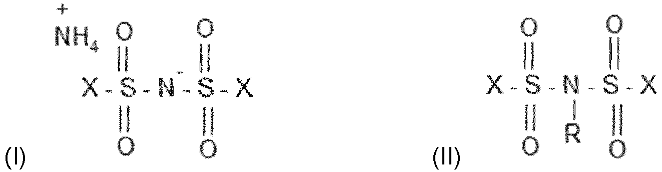 wherein
each of X is independently selected from F, Cl and Br, and
R is a linear or branched alkyl group comprising from 1 to 10 carbon atoms; said method comprising:
   a) providing a sulfuryl halogenide of formula XₐSO₂X_{b} wherein each of Xₐ and X_{b}, identical or different from each other, is selected from F, Cl and Br;
   b) providing at least one ammonium salt;
   c) contacting said sulfuryl halogenide and said at least one ammonium salt, to obtain the bis(halogeno sulfonyl)imide of formula (I) or (II).

Preferably, in the sulfuryl halogenide of formula XₐSO₂X_{b}, Xₐ and X_{b} are identical to each other.

Preferably, the sulfuryl halogenide is selected from Cl-SO₂-Cl, Cl-SO₂-F and F-SO₂-F; more preferably it is Cl-SO₂-Cl.

Preferably, Cl-SO₂-Cl is provided in its liquid form.

Preferably, each of Cl-SO₂-F and F-SO₂-F is provided in its gas form.

Preferably, said at least one ammonium salt complies with formula (III): 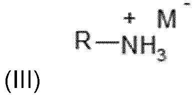 wherein
R is a linear or branched alkyl group comprising from 1 to 10 carbon atoms, and
M anion is selected in the group comprising, preferably consisting of: F, Cl, carboxylate, sulfate, hydrogen-sulfate, carbonate, hydrogen-carbonate, tetrafluoroborate and hexafluorophosphate.

Preferably, said at least one ammonium salt is provided in its solid form.

Preferably, said ammonium salt is NH₄Cl or NH₄F.

Under step c), the sulfuryl halogenide reacts with the at least one ammonium salt to obtain the bis(halogeno sulfonyl)imide of formula (I) or (II) as defined above.

Step c) can be performed in the presence or in the absence of a solvent.

When step c) is performed in the presence of a solvent, said solvent is preferably selected in the group comprising: ethylene carbonate, propylene carbonate, butylene carbonate, γ-butyrolactone, γ-valerolactone, dimethoxymethane, 1,2-dimethoxyethane, tetrahydrofuran, 2-methyltetrahydrofuran, 1,3-dioxane, 4-methyl-1,3- dioxolane, methyl formate, methyl acetate, methyl propionate, dimethyl carbonate, ethyl methyl carbonate, diethyl carbonate, sulfolane, 3-methyl sulfolane, dimethylsulfoxide, N,N-dimethylformamide, N-methyl oxazolidinone, acetonitrile, valeronitrile, benzonitrile, ethyl acetate, isopropyl acetate, n-butyl acetate, nitromethane and nitrobenzene.

When step c) is performed in the absence of a solvent, the sulfuryl halogenide is advantageously in its liquid form. According to this embodiment, when Cl-SO₂-F and F-SO₂-F are used, they are liquefied under pressure before performing step c).

Preferably, step c) is performed at a temperature from about 15°C to about 150°C.

Preferably, step c) is performed under stirring.

According to an embodiment, step c) is performed in the presence of a solvent.

According to this embodiment, the temperature is properly selected based on the solvent used and its boiling temperature. For example, the temperature can be kept between 15°C and 100°C, more preferably between 15°C and 75°C and even more preferably between 20°C and 50°C.

Also, according to this embodiment, the molar ratio between the sulfuryl halogenide and said at least one ammonium salt in step c) is between 100 to 0.1 , preferably between 10 to 1, more preferably between 1.5 to 1.

According to another embodiment, step c) is performed in the absence of any solvent (also referred to as "neat conditions").

According to this embodiment, the temperature is between 50°C and 100°C, more preferably between 60°C and 70°C.

Also, according to this embodiment, the molar ratio between the sulfuryl halogenide and said at least one ammonium salt is between 100 to 0.1, more preferably between 50 to 1, and even more preferably between 5 to 1.

When the sulfuryl halogenide is used in excess as described in any one of the above embodiments, the unreacted amount of the sulfuryl halogenide can be advantageously distilled off. More preferably, such distilled amount of sulfuryl halogenide is recycled, i.e. provided under step a).

Preferably, in the method according to the present invention, the sulfuryl halogenide is ClSO₂Cl and the at least one ammonium salt is NH₄⁺ Cl⁻.

According to such embodiment, the method according to the present invention comprises:
a*) providing a sulfuryl halogenide of formula ClSO₂Cl,
b) providing at least one ammonium salt of formula NH₄⁺ Cl⁻ ,
c*) contacting said sulfuryl halogenide and said at least one ammonium salt, to obtain the bis(halogeno sulfonyl)imide of formula (I) wherein each of X is chlorine [ammonium-CSl].

The reaction in step c*) is advantageously performed under the conditions disclosed above for step c).

Ammonium-CSI can be isolated or purified from the reaction environment. As an alternative, it can be directly used in a subsequent reaction.

Advantageously, ammonium-CSI can be reacted with a fluorine-containing compound, to manufacture ammonium-FSI.

According to this embodiment, the method according to the present invention comprises after step c*), a step d) of contacting said ammonium-CSI with at least one fluorinating agent, so as to obtain ammonium-FSI.

Step d) hence encompasses a fluorination reaction of said ammonium-CSI to obtain ammonium-FSI.

Such fluorinating agent is preferably selected in the group comprising: HF, more preferably anhydrous HF, or X_{c}F wherein X_{c} is selected from NH₄, Cs, Li, K.

When the fluorinating agent is NH₄F, the reaction proceeds generating NH₄Cl as a side-product, which can be advantageously recycled, by providing it under step b).

Preferably, in the method according to the present invention, the sulfuryl halogenide is ClSO₂Cl and the at least one ammonium salt is NH₄⁺ F⁻.

According to this embodiment, the method according to the present invention comprises:
a**) providing a sulfuryl halogenide of formula ClSO₂Cl;
b) providing at least one ammonium salt of formula NH₄⁺ F⁻;
c**) contacting said sulfuryl halogenide and said at least one ammonium salt, to obtain the bis(halogeno sulfonyl)imide of formula (I) wherein each of X is fluorine [ammonium-FSI].

The reaction in step c**) is advantageously performed under the conditions disclosed above for step c).

The ammonium-FSI obtained at the end of step d) or of step c**) can be isolated or purified. As an alternative, such ammonium-FSI can be directly used in a subsequent reaction.

The ammonium-FSI obtained at the end of step d) or of step c**) can be advantageously used as an intermediate compound in the manufacture of LiFSI.

According to a preferred embodiment, NH₄-FSI is reacted with a lithiation agent to manufacture LiFSI.

Thus, in a further embodiment, the present invention relates to a method for manufacturing LiFSI, said method comprising after step d) or step c**), step e) of contacting said ammonium-FSI and a compound of formula LiX_{d}, thus obtaining LiFSI.

Preferably, said compound LiX_{d} is selected from the group consisting of lithium chloride (LiCl), lithium fluoride (LiF), lithium carbonate (Li₂CO₃), lithium hydroxide optionally in its hydrate form (LiOH, LiOH.H₂O) lithium sulfate (Li₂SO₄), lithium carboxylate (Liₙ(RCO₂)ₙ), Li₂SiO₃, Li₂B₄O₇ and mixtures thereof.

Preferably, step e) is performed in the presence of at least one solvent, such that LiFSI is obtained as a liquid composition.

Preferably, said at least one solvent is selected in the group comprising ethylene carbonate, propylene carbonate, butylene carbonate, γ-butyrolactone, γ-valerolactone, dimethoxymethane, 1,2-dimethoxyethane, tetrahydrofuran, 2-methyltetrahydrofuran, 1,3-dioxane, 4-methyl-1,3-dioxolane, methyl formate, methyl acetate, methyl propionate, dimethyl carbonate, ethyl methyl carbonate, diethyl carbonate, sulfolane, 3-methyl sulfolane, dimethylsulfoxide, N,N-dimethylformamide, N-methyl oxazolidinone, acetonitrile, valeronitrile, benzonitrile, ethyl acetate, isopropyl acetate, n-butyl acetate, nitromethane and nitrobenzene.

More preferably, said solvent is selected from ethylene carbonate, propylene carbonate, butylene carbonate, tetrahydrofuran, dimethyl carbonate, ethyl methyl carbonate, diethyl carbonate, ethyl acetate, isopropyl acetate and n-butyl acetate, even more preferred solvents include dimethyl carbonate, ethyl methyl carbonate, diethyl carbonate, ethyl acetate, isopropyl acetate and n-butyl acetate. Even more preferably, said solvent is selected from ethyl methyl carbonate and n-butyl acetate.

More preferably, said liquid composition comprises from 1 to 70 wt.%, even more preferably from 5 to 50 wt.% and still more preferably from 15 to 40 wt.% of said LiFSI based on the total weight of said liquid composition.

The method of the present invention is performed in a reactor.

Preferably, some of the steps, or more preferably all steps, of the method according to the invention are carried out in a reactor capable of withstanding the corrosion of the reagents and of the products and/or by-products obtained as the reaction proceeds. For this purpose, corrosion-resistant materials are selected for the part of the reactor in contact with the reaction media.

Preferably, said corrosion-resistant material is selected from alloys based on molybdenum, chromium, cobalt, iron, copper, manganese, titanium, zirconium, aluminum, carbon and tungsten, commercially available under the trade name Hastelloy^{®}, such as in particular Hastelloy^{®} C276; alloys of nickel, chromium, iron and manganese to which copper and/or molybdenum are added, commercially available under the trade name Inconel^{®} or Monel^{™}, such as in particular Inconel^{®} 600, 625 or 718.

Said corrosion-resistant material can be selected from stainless steels, such as austenitic steels and more particularly the 304, 304L, 316 or 316L stainless steels. Preferably, a steel having a nickel content of at most 22 wt.%, preferably of between 6 wt.% and 20 wt.% and more preferentially of between 8 wt.% and 14 wt.%, is used. The 304 and 304L steels have a nickel content that varies between 8 wt.% and 12 wt.%, and the 316 and 316L steels have a nickel content that varies between 10 wt.% and 14 wt.%. More preferably, 316L steels are chosen.

Said corrosion-resistant material can be a polymeric compound resistant to the corrosion of the reaction medium, which provides a coating onto the part of the reactor in contact with the reaction media. For example, mention can be made of PTFE (polytetrafluoroethylene or Teflon) or PFA (perfluoroalkyl resins).

As an alternative and more preferably, said anti-corrosion material is selected from glass, glass-lined and enamel equipment.

Preferably and advantageously, all materials used in the method according to the invention, including reactants, may preferably show very high purity.

Preferably, their content of metal components such as Na, K, Ca, Mg, Fe, Cu, Cr, Ni, Zn, is below 10 ppm, more preferably below 5 ppm, or below 2 ppm.

In a further object, the present invention relates to the use of said liquid composition as a non-aqueous electrolyte solution in a battery.

Should the disclosure of any patents, patent applications, and publications which are incorporated herein by reference conflict with the description of the present application to the extent that it may render a term unclear, the present description shall take precedence.

The present invention will be now described in more detail with reference to the following examples, whose purpose is merely illustrative and not intended to limit the scope of the disclosure.

### EXAMPLES

### Materials

Sulfuryl chloride, acetonitrile and ammonium chloride were purchased and used as such.
SO₂Cl₂ : Sigma Aldrich (product code #157767, batch #STBK3652)
NH₄Cl : Alfa Alesar (product code #12361, batch #Y28E029)

### Methods

¹⁵N-NMR analysis was performed using Insensitive Nuclear Enhancement by Polarization Transfer (INEPT) as a signal enhancement method.

### Example 1 (neat conditions)

In an oven-dried 25 mL round bottom flask containing a PTFE-coated magnetic stirrer, ammonium chloride (1.6 g, 30 mmol, 0.3 equiv.) was added portionwise to neat sulfuryl chloride (8.08 mL, 100 mmol, 1 equiv.) under stirring, at 0 °C. The flask was equipped with a reflux condenser and the reaction mixture was stirred at reflux for 24 hours. The reaction mixture was then cooled down to room temperature, filtered and concentrated under reduced pressure to give an orange solid.

Trituration in acetonitrile finally afforded the pure product as a white solid.

Yield was 44% (1.54 g, 6.6 mmol) and the characteristic peaks were found with ¹H-NMR and ¹⁵N-NMR analysis.

### Example 2 (with acetonitrile as the solvent)

Following the procedure disclosed in Eample 1 above, an excess of sulfuryl chloride was reacted with ammonium chloride, but in the presence of acetonitrile as the solvent.

The reaction medium was filtered to remove ammonium chloride, and a mixture was obtained, which was analyzed via ¹H-NMR.

This compound was further analyzed by ¹H-NMR and ¹⁵N-NMR. No peak was detected for HCSI or other side products.

### Example 3 (neat conditions)

Following the same procedure disclosed in Example 1 above, SO₂Cl₂ kept at its boiling point was reacted with a sub-stoichiometric amount of NH₄Cl.

After 1 hour at 69 °C, the reaction medium started to solidify and at the end of 24 hours reaction, a mixture of ammonium-CSI and unreacted solid NH₄Cl was recovered.

Ammonium-CSI was obtained at a yield of 48 %. The product was analyzed and characterized by ¹H-NMR and ¹⁵N-NMR.

## Claims

1. A method for manufacturing bis(halogeno sulfonyl)imide of formula (I) or (II): 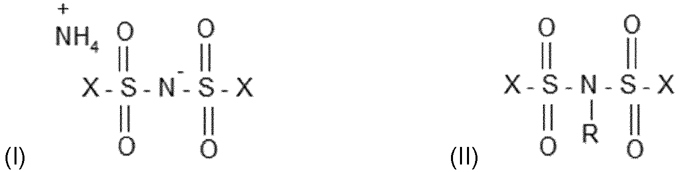 wherein
R is a linear or branched alkyl group comprising from 1 to 10 carbon atoms and each of X is independently selected from F, Cl and Br;
said method comprising:
a) providing a sulfuryl halogenide of formula XₐSO₂X_{b}
wherein each of Xₐ and X_{b}, identical or different from each other, is selected from F, Cl and Br;
b) providing at least one ammonium salt;
c) contacting said sulfuryl halogenide and said at least one ammonium salt, to obtain the bis(halogeno sulfonyl)imide of formula (I) or (II).

2. The method according to Claim 1, wherein the sulfuryl halogenide is selected from Cl-SO₂-Cl, Cl-SO₂-F and F-SO₂-F.

3. The method according to Claim 1 or 2, wherein said at least one ammonium salt complies with formula (III): 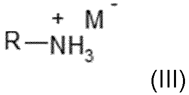 wherein
R is H or a linear or branched alkyl group comprising from 1 to 10 carbon atoms, and
M is selected in the group comprising: F, Cl, carboxylate, sulfate, hydrogen-sulfate, carbonate, hydrogen-carbonate, tetrafluoroborate, hexafluorophosphate.

4. The method according to any one of the preceding Claims, wherein step c) is performed at a temperature from 15°C to 150°C and/or under stirring.

5. The method according to any one of the preceding Claims, wherein step c) is performed in the presence of a solvent.

6. The method according to Claim 5, wherein said solvent is selected in the group comprising: ethylene carbonate, propylene carbonate, butylene carbonate, γ-butyrolactone, γ-valerolactone, dimethoxymethane, 1,2-dimethoxyethane, tetrahydrofuran, 2-methyltetrahydrofuran, 1,3-dioxane, 4-methyl-1,3- dioxolane, methyl formate, methyl acetate, methyl propionate, dimethyl carbonate, ethyl methyl carbonate, diethyl carbonate, sulfolane, 3-methyl sulfolane, dimethylsulfoxide, N,N-dimethylformamide, N-methyl oxazolidinone, acetonitrile, valeronitrile, benzonitrile, ethyl acetate, isopropyl acetate, n-butyl acetate, nitromethane and nitrobenzene.

7. The method according to Claim 5 or 6, wherein step c) is performed with a molar ratio between the sulfuryl halogenide and said at least one ammonium salt between 100 to 0.1, preferably 10 to 1, more preferably between 1.5 to 1.

8. The method according to any one of Claim 1 to 4, wherein step c) is performed in the absence of a solvent and/or a temperature between 50°C and 100°C, more preferably between 60°C and 70°C.

9. The method according to any one of the preceding claims, wherein the molar ratio between the sulfuryl halogenide and said at least one ammonium salt is between 100 to 0.1, preferably between 50 to 1, and more preferably between 5 to 1.

10. The method according to any one of the preceding claims, wherein in the bis(halogeno sulfonyl)imide of formula (I) each of X is chlorine [ammonium-CSI] and the method comprises:
a*) providing a sulfuryl halogenide of formula ClSO₂Cl;
b) providing at least one ammonium salt of formula NH₄⁺ Cl⁻;
c*) contacting said sulfuryl halogenide and said at least one ammonium salt, thus obtaining the ammonium-CSI.

11. The method according to Claim 10, said method comprising after step c*), step d) of contacting said ammonium-CSI with at least one fluorinating agent, so as to obtain ammonium-FSI.

12. The method according to Claim 11, wherein said at least one fluorinating agent is selected in the group comprising: HF, more preferably anhydrous HF, or X_{c}F wherein X_{c} is selected from NH₄, Cs, Li, K.

13. The method according to any one of Claims 1 to 9, wherein in the bis(halogeno sulfonyl)imide of formula (I) each of X is fluorine [ammonium-FSI] and the method comprises:
a**) providing a sulfuryl halogenide of formula ClSO₂Cl;
b) providing at least one ammonium salt of formula NH₄⁺ F⁻;
c**) contacting said sulfuryl halogenide and said at least one ammonium salt, thus obtaining the ammonium-FSI.

14. A method for manufacturing lithium bis(fluoro sulfonyl) imide (LiFSI), said method comprising, after step d) as defined in Claim 11 or after step c**) as defined in Claim 13, step e) of contacting the ammonium-FSI and a compound of formula LiX_{d}, thus obtaining LiFSI.

15. The method according to Claim 14, wherein said compound of formula LiX_{d} is selected from the group consisting of LiCl, LiF, Li₂CO₃, LiOH, LiOH.H₂O, Li₂SO₄, Liₙ(RCO₂)ₙ, Li₂SiO₃, Li₂B₄O₇ and mixtures thereof.

16. The method according to Claim 14 or 15, wherein step e) is performed in the presence of at least one solvent and LiFSi is obtained as liquid composition comprising from 1 to 70 wt.% of LiFSI based on the total weight of said liquid composition.

## Patentansprüche

1. Verfahren zur Herstellung von Bis(halogensulfonyl)imid der Formel (I) oder (II): 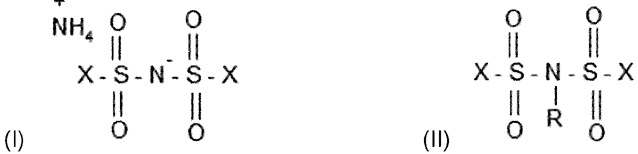 wobei
R für eine lineare oder verzweigte Alkylgruppe mit 1 bis 10 Kohlenstoffatomen steht und X jeweils unabhängig aus F, Cl und Br ausgewählt ist;
wobei das Verfahren Folgendes umfasst:
a) Bereitstellen eines Sulfurylhalogenids der Formel XₐSO₂X_{b}
wobei Xₐ und X_{b} jeweils gleich oder voneinander verschieden aus F, Cl und Br ausgewählt sind;
b) Bereitstellen mindestens eines Ammoniumsalzes;
c) Inkontaktbringen des Sulfurylhalogenids und des mindestens einen Ammoniumsalzes zum Erhalt des Bis(halogensulfonyl)imids der Formel (I) oder (II).

2. Verfahren nach Anspruch 1, wobei das Sulfurylhalogenid aus Cl-SO₂-Cl, Cl-SO₂-F und F-SO₂-F ausgewählt wird.

3. Verfahren nach Anspruch 1 oder 2, wobei das mindestens eine Ammoniumsalz der Formel (III) entspricht: 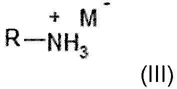 wobei
R für H oder eine lineare oder verzweigte Alkylgruppe mit 1 bis 10 Kohlenstoffatomen steht und
M ausgewählt ist aus der Gruppe umfassend: F, Cl, Carboxylat, Sulfat, Hydrogensulfat, Carbonat, Hydrogencarbonat, Tetrafluoroborat, Hexafluorophosphat.

4. Verfahren nach einem der vorhergehenden Ansprüche, wobei Schritt c) bei einer Temperatur von 15 °C bis 150 °C und/oder unter Rühren durchgeführt wird.

5. Verfahren nach einem der vorhergehenden Ansprüche, wobei Schritt c) in Gegenwart eines Lösungsmittels durchgeführt wird.

6. Verfahren nach Anspruch 5, wobei das Lösungsmittel aus der Gruppe umfassend Ethylencarbonat, Propylencarbonat, Butylencarbonat, γ-Butyrolacton, γ-Valerolacton, Dimethoxymethan, 1,2-Dimethoxyethan, Tetrahydrofuran, 2-Methyltetrahydrofuran, 1,3-Dioxan, 4-Methyl-1,3-dioxolan, Ameisensäuremethylester, Essigsäuremethylester, Propionsäuremethylester, Dimethylcarbonat, Ethylmethylcarbonat, Diethylcarbonat, Sulfolan, 3-Methylsulfolan, Dimethylsulfoxid, N,N-Dimethylformamid, N-Methyloxazolidinon, Acetonitril, Valeronitril, Benzonitril, Essigsäureethylester, Essigsäureisopropylester, Essigsäure-n-butylester, Nitromethan und Nitrobenzol ausgewählt wird.

7. Verfahren nach Anspruch 5 oder 6, wobei Schritt c) mit einem Molverhältnis zwischen dem Sulfurylhalogenid und dem mindestens einen Ammoniumsalz zwischen 100 bis 0,1, vorzugsweise 10 bis 1, weiter bevorzugt zwischen 1,5 bis 1, durchgeführt wird.

8. Verfahren nach einem der Ansprüche 1 bis 4, wobei Schritt c) in Abwesenheit eines Lösungsmittels und/oder bei einer Temperatur zwischen 50 °C und 100 °C, weiter bevorzugt zwischen 60 °C und 70 °C, durchgeführt wird.

9. Verfahren nach einem der vorhergehenden Ansprüche, wobei das Molverhältnis zwischen dem Sulfurylhalogenid und dem mindestens einen Ammoniumsalz zwischen 100 bis 0,1, vorzugsweise zwischen 50 bis 1 und weiter bevorzugt zwischen 5 bis 1 liegt.

10. Verfahren nach einem der vorhergehenden Ansprüche, wobei in dem Bis(halogensulfonyl)imid der Formel (I) X jeweils für Chlor steht [Ammonium-CSI] und das Verfahren Folgendes umfasst:
a*) Bereitstellen eines Sulfurylhalogenids der Formel ClSO₂Cl;
b) Bereitstellen mindestens eines Ammoniumsalzes der Formel NH₄⁺ Cl⁻;
c*) Inkontaktbringen des Sulfurylhalogenids und des mindestens einen Ammoniumsalzes, wodurch das Ammonium-CSI erhalten wird.

11. Verfahren nach Anspruch 10, wobei das Verfahren nach Schritt c*) den Schritt d) des Inkontaktbringens des Ammonium-CSI mit mindestens einem Fluorierungsmittel zum Erhalt von Ammonium-FSI umfasst.

12. Verfahren nach Anspruch 11, wobei das mindestens eine Fluorierungsmittel aus der Gruppe ausgewählt wird, die Folgendes umfasst: HF, weiter bevorzugt wasserfreies HF, oder X_{c}F, wobei X_{c} aus NH₄, Cs, Li, K ausgewählt ist.

13. Verfahren nach einem der Ansprüche 1 bis 9, wobei in dem Bis(halogensulfonyl)imid der Formel (I) X jeweils für Fluor steht [Ammonium-FSI] und das Verfahren Folgendes umfasst:
a**) Bereitstellen eines Sulfurylhalogenids der Formel ClSO₂Cl;
b) Bereitstellen mindestens eines Ammoniumsalzes der Formel NH₄⁺ F⁻;
c**) Inkontaktbringen des Sulfurylhalogenids und des mindestens einen Ammoniumsalzes, wodurch das Ammonium-FSI erhalten wird.

14. Verfahren zur Herstellung von Lithiumbis(fluorsulfonyl)imid (LiFSI), wobei das Verfahren nach Schritt d) gemäß Anspruch 11 oder nach Schritt c**) gemäß Anspruch 13 den Schritt e) des Inkontaktbringens des Ammonium-FSI und einer Verbindung der Formel LiX_{d} umfasst, wodurch LiFSI erhalten wird.

15. Verfahren nach Anspruch 14, wobei die Verbindung der Formel LiX_{d} aus der Gruppe bestehend aus LiCl, LiF, Li₂CO₃, LiOH, LiOH.H₂O, Li₂SO₄, Liₙ(RCO₂)ₙ, Li₂SiO₃, Li₂B₄O₇ und Mischungen davon ausgewählt ist.

16. Verfahren nach Anspruch 14 oder 15, wobei Schritt e) in Gegenwart mindestens eines Lösungsmittels durchgeführt wird und LiFSi als flüssige Zusammensetzung erhalten wird, die 1 bis 70 Gew.-% LiFSI, bezogen auf das Gesamtgewicht der flüssigen Zusammensetzung, umfasst.

## Revendications

1. Procédé de fabrication de bis(halogénosulfonyl)imide de formule (I) ou (II) : 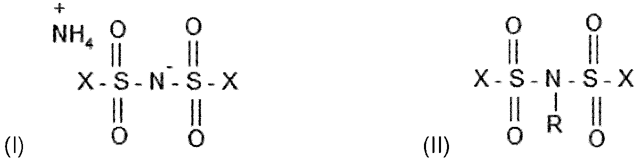 où
R est un groupe alkyle linéaire ou ramifié comprenant de 1 à 10 atomes de carbone et chacun des X est indépendamment choisi parmi F, Cl et Br ;
ledit procédé comprenant :
a) la mise à disposition d'un halogénure de sulfuryle de formule XₐSO₂X_{b}
où chacun de Xₐ et X_{b}, identique à l'autre ou différent de l'autre, est choisi parmi F, Cl et Br ;
b) la mise à disposition d'au moins un sel d'ammonium ;
c) la mise en contact dudit halogénure de sulfuryle et dudit au moins un sel d'ammonium, pour obtenir le bis(halogénosulfonyl)imide de formule (I) ou (II).

2. Procédé selon la revendication 1, dans lequel l'halogénure de sulfuryle est choisi parmi Cl-SO₂-Cl, Cl-SO₂-F et F-SO₂-F.

3. Procédé selon la revendication 1 ou 2, dans lequel ledit au moins un sel d'ammonium répond à la formule (III) : 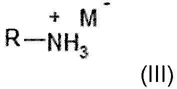 où
R est H ou un groupe alkyle linéaire ou ramifié comprenant de 1 à 10 atomes de carbone et
M est choisi dans le groupe comprenant : F, Cl, carboxylate, sulfate, hydrogénosulfate, carbonate, hydrogénocarbonate, tétrafluoroborate, hexafluorophosphate.

4. Procédé selon l'une quelconque des revendications précédentes, dans lequel l'étape c) est effectuée à une température allant de 15 °C à 150 °C et/ou sous agitation.

5. Procédé selon l'une quelconque des revendications précédentes, dans lequel l'étape c) est effectuée en présence d'un solvant.

6. Procédé selon la revendication 5, dans lequel ledit solvant est choisi dans le groupe comprenant : le carbonate d'éthylène, le carbonate de propylène, le carbonate de butylène, la γ-butyrolactone, la γ-valérolactone, le diméthoxyméthane, le 1,2-diméthoxyéthane, le tétrahydrofurane, le 2-méthyltétrahydrofurane, le 1,3-dioxane, le 4-méthyl-1,3-dioxolane, le formiate de méthyle, l'acétate de méthyle, le propionate de méthyle, le carbonate de diméthyle, le carbonate d'éthyle et de méthyle, le carbonate de diéthyle, le sulfolane, le 3-méthylsulfolane, le diméthylsulfoxyde, le N,N-diméthylformamide, la N-méthyloxazolidinone, l'acétonitrile, le valéronitrile, le benzonitrile, l'acétate d'éthyle, l'acétate d'isopropyle, l'acétate de n-butyle, le nitrométhane et le nitrobenzène.

7. Procédé selon la revendication 5 ou 6, dans lequel l'étape c) est effectuée avec un rapport molaire entre l'halogénure de sulfuryle et ledit au moins un sel d'ammonium compris entre 100 à 0,1, de préférence entre 10 à 1, plus préférablement entre 1,5 à 1.

8. Procédé selon l'une quelconque des revendications 1 à 4, dans lequel l'étape c) est effectuée en l'absence d'un solvant et/ou à une température comprise entre 50 °C et 100 °C, plus préférablement entre 60 °C et 70 °C.

9. Procédé selon l'une quelconque des revendications précédentes, dans lequel le rapport molaire entre l'halogénure de sulfuryle et ledit au moins un sel d'ammonium est compris entre 100 à 0,1, de préférence entre 50 à 1 et plus préférablement entre 5 à 1.

10. Procédé selon l'une quelconque des revendications précédentes, dans lequel, dans le bis (halogénosulfonyl)imide de formule (I), chacun des X est le chlore [CSI d'ammonium] et le procédé comprenant :
a*) la mise à disposition d'un halogénure de sulfuryle de formule ClSO₂Cl ;
b) la mise à disposition d'au moins un sel d'ammonium de formule NH₄⁺Cl⁻ ;
c*) la mise en contact dudit halogénure de sulfuryle et dudit au moins un sel d'ammonium, ce qui permet d'obtenir le CSI d'ammonium.

11. Procédé selon la revendication 10, ledit procédé comprenant, après l'étape c*), l'étape d) de mise en contact dudit CSI d'ammonium avec au moins un agent de fluoration, de manière à obtenir du FSI d'ammonium.

12. Procédé selon la revendication 11, dans lequel ledit au moins un agent de fluoration est choisi dans le groupe comprenant : HF, plus préférablement HF anhydre, ou X_{c}F où X_{c} est choisi parmi NH₄, Cs, Li, K.

13. Procédé selon l'une quelconque des revendications 1 à 9, dans lequel, dans le bis(halogénosulfonyl)imide de formule (I), chacun des X est le fluor [FSI d'ammonium] et le procédé comprenant :
a**) la mise à disposition d'un halogénure de sulfuryle de formule ClSO₂Cl ;
b) la mise à disposition d'au moins un sel d'ammonium de formule NH₄⁺F⁻ ;
c**) la mise en contact dudit halogénure de sulfuryle et dudit au moins un sel d'ammonium, ce qui permet d'obtenir le FSI d'ammonium.

14. Procédé de fabrication de bis(fluorosulfonyl)imidure de lithium (LiFSI), ledit procédé comprenant, après l'étape d) telle que définie dans la revendication 11 ou après l'étape c**) telle que définie dans la revendication 13, l'étape e) de mise en contact du FSI d'ammonium et d'un composé de formule LiX_{d}, ce qui permet d'obtenir LiFSI.

15. Procédé selon la revendication 14, dans lequel ledit composé de formule LiX_{d} est choisi dans le groupe constitué par LiCl, LiF, Li₂CO₃, LiOH, LiOH.H₂O, Li₂SO₄, Liₙ(RCO₂)ₙ, Li₂SiO₃, Li₂B₄O₇ et les mélanges de ceux-ci.

16. Procédé selon la revendication 14 ou 15, dans lequel l'étape e) est effectuée en présence d'au moins un solvant et LiFSI est obtenu sous forme de composition liquide comprenant de 1 à 70 % en poids de LiFSI sur la base du poids total de ladite composition liquide.
